# EUROPEAN PATENT SPECIFICATION

(11) **EP 4 431 515 B1**
(45) Date of publication and mention of the grant of the patent: **29.07.2026**
(21) Application number: 22889458.0
(22) Date of filing: 07.11.2022
(51) Int. Cl.: C07F 5/02, A61K 31/69, A61P 35/00

(54) **NOVEL CRYSTAL FORMS OF PEPTIDE BORIC ACID COMPOUND AND PREPARATION METHODS THEREFOR**
NEUE KRISTALLFORMEN EINER PEPTIDBORSÄUREVERBINDUNG UND HERSTELLUNGSVERFAHREN DAFÜR
NOUVELLES FORMES CRISTALLINES D'UN COMPOSÉ D'ACIDE BORIQUE PEPTIDIQUE ET LEURS PROCÉDÉS DE PRÉPARATION

(30) Priority: 08.11.2021 CN 202111310759
(43) Date of publication of application: 18.09.2024
(73) Proprietor: Jiangsu Chuangte Pharmaceutical Technology Co., Ltd., Yancheng, Jiangsu 224199 (CN)
(72) Inventor: ZHU, Yongqiang, Yancheng, Jiangsu 224199 (CN); CHEN, Qi, Yancheng, Jiangsu 224199 (CN); LIU, Jia, Yancheng, Jiangsu 224199 (CN); LI, Cancan, Yancheng, Jiangsu 224199 (CN); YANG, Yang, Yancheng, Jiangsu 224199 (CN)
(74) Representative: Engelhard, Markus
(86) International application number: PCT/CN2022/130193
(87) International publication number: WO 2023/078437

(56) References cited:
- WO-A1-2019/228299
- CN-A- 110 540 547
- STEPHEN BYRN ET AL: "Pharmaceutical Solids: A strategic Approach to Regulatory Considerations", PHARMACEUTICAL RESEARCH, SPRINGER BERLIN HEIDELBERG, BERLIN/HEIDELBERG, vol. 12, no. 7, 1 July 1995 (1995-07-01), pages 945 - 954, XP000996386, ISSN: 0724-8741, DOI: 10.1023/A:1016241927429
- HILFIKER R (EDITOR) ED - HILFIKER R: "Polymorphism in the Pharmaceutical Industry", 1 January 2006, 20060101, PAGE(S) 1 - 19, ISBN: 978-3-527-31146-0, XP002528052
- MINO R CAIRA ED - MONTCHAMP JEAN-LUC: "CRYSTALLINE POLYMORPHISM OF ORGANIC COMPOUNDS", TOPICS IN CURRENT CHEMISTRY; [TOPICS IN CURRENT CHEMISTRY], SPRINGER, BERLIN, DE, vol. 198, 1 January 1998 (1998-01-01), pages 163 - 208, XP001156954, ISSN: 0340-1022, [retrieved on 19990226], DOI: 10.1007/3-540-69178-2_5
- LEI MENG, FENG HUAYUN, BAI ENHE, ZHOU HUI, WANG JIA, QIN YANRU, ZHANG HAOYANG, WANG XUEYUAN, LIU ZHAOGANG, HAI OU, LIU JIA, ZHU YO: "Discovery of a novel dipeptidyl boronic acid proteasome inhibitor for the treatment of multiple myeloma and triple-negative breast cancer", ORGANIC & BIOMOLECULAR CHEMISTRY, ROYAL SOCIETY OF CHEMISTRY, vol. 17, no. 3, 16 January 2019 (2019-01-16), pages 683 - 691, XP093062862, ISSN: 1477-0520, DOI: 10.1039/C8OB02668H

## Description

### TECHNICAL FIELD

The present invention relates to crystal forms of a pharmaceutical compound, and more specifically to three novel crystal forms A, B, and D of *N*-((*R*)-1-(((*R*)-1-(1,3,6,2-dioxazaborolan-2-yl)-3-methylbutyl)amino)-3-(methylthio)-1-oxoprop-2-yl)-2,5-dichlorobenzamide and methods for preparing the novel crystal forms.

### BACKGROUND ART

Ubiquitin-proteasome pathway (UPP) is the major pathway for the degradation of intracellular protein systems and is involved in many physiologically important cellular processes, including signal transduction, immune responses, unfolded protein responses, and cell cycle progression. This pathway is closely related to the development of cardiovascular and cerebrovascular diseases, cancers, and nervous system degenerative diseases. Proteasome inhibitors have been used for treating disease, and the first proteasome inhibitor was approved for marketing in 2003.

Currently, representative proteasome inhibitors used clinically are bortezomib, carfilzomib, and ixazomib. Bortezomib and carfilzomib are administered by intravenous injection, which needs to be performed by professional medical workers, so that the compliance and the quality of life of patients are inevitably reduced; besides, the two drugs exhibit such problems in safety and effectiveness as peripheral neurotoxicity, cardiotoxicity, and drug resistance in clinical application. Ixazomib, although being an oral preparation, has poor clinical effectiveness and needs to be administered in combination with other drugs. Therefore, there is a need to develop a novel oral proteasome inhibitor with high effectiveness and low toxicity.

The patent CN112384519 discloses *N*-((*R*)-1-(((*R*)-1-(1,3,6,2-dioxazaborolan-2-yl)-3-methylbutyl) amino)-3-(methylthio)-1-oxoprop-2-yl)-2,5-dichlorobenzamide as a proteasome inhibitor for the treatment of solid and hematological tumors, and the structure of the compound is shown as formula (I) below.

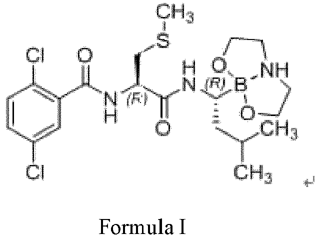

Organic & Biomolecular Chemistry, Royal Society of Chemistry, vol. 17, no. 3, 2019, pages 683-691 discloses compound 8t and a method for its preparation.

Different crystal forms of the same compound may change the physicochemical properties of the compound, e.g., resulting in different solubility, thermodynamic stability, and density or melting points of the different forms. For a compound with the potential of being developed into a drug, these properties may directly affect the handling or production of the compound as a drug substance and preparation, and such physicochemical properties as stability, solubility, and bioavailability of the preparation may be affected, which may have a significant impact on the efficacy or bioavailability of the active ingredient. Therefore, it is of great significance to obtain a stable crystal form of a compound.

### SUMMARY

The present invention mainly provides novel crystal forms of *N*-((*R*)-1-(((*R*)-1-(1,3,6,2-dioxazaborolan-2-yl)-3-methylbutyl)amino)-3-(methylthio)-1-oxoprop-2-yl)-2,5-dichlorobenzami de and preparation methods therefor, specifically as follows:
Provided is a crystal form A of *N*-((*R*)-1-(((R)-1-(1,3,6,2-dioxazaborolan-2-yl)-3-methylbutyl) amino)-3-(methylthio)-1-oxoprop-2-yl)-2,5-dichlorobenzamide, wherein the X-ray powder diffraction pattern of the crystal form A has characteristic peaks at the following diffraction angles 2θ: 9.09, 11.30, 14.27, 17.00, 17.61, 20.28, 23.75, 24.85, and 25.25, the measurement error of 2θ being ±0.2.

Provided is a method for preparing the crystal form A, which is as follows:
step (1): dissolving an organic base in an organic solvent I and heating to 70-75 °C;
step (2): adding ((*R*)-1-((*R*)-2-(2,5-dichlorobenzamido)-3-(methylthio)propionamido)-3-methylbutyl)boric acid/organic solvent I solution dropwise to the solution obtained in step (1) while maintaining the temperature at 70-75 °C;
step (3): after the dropwise addition, cooling to 60 °C, stirring for 2 h, cooling to 20-25 °C, stirring for 8 h, filtering, and drying to obtain a eutectic solid;
step (4): dissolving the eutectic solid in an organic solvent II, stirring for 2 h at 25-35 °C, heating to 55-65 °C and stirring for 2 h with a heating rate of 15 °C/h, stirring for 2 h at 25-35 °C with a cooling rate of 15 °C/h, and crystallizing;
step (5): filtering and drying to obtain the crystal form A;

wherein, the organic base is selected from diethanolamine, ethanolamine, or ethylenediamine;
the solvent I or II is selected from ethyl acetate, dichloromethane, ketone solvents, ether solvents, alcohol solvents, nitrile solvents, or mixtures thereof;
the volume-to-mass ratio of the solvent I to the organic base is 15-45:1 and is expressed in mL/g;
the volume-to-mass ratio of the solvent II to the eutectic solid is 5-15:1 and is expressed in mL/g;

The volume-to-mass ratio of the organic solvent I solution to ((*R*)-1-((*R*)-2-(2,5-dichlorobenzamido)-3-(methylthio)propionamido)-3-methylbutyl)boric acid in the ((*R*)-1-((*R*)-2-(2,5-dichlorobenzamido)-3-(methylthio)propionamido)-3-methylbutyl)boric acid/organic solvent I solution is 1-5:1 and is expressed in mL/g.

Provided is a crystal form B of *N*-((*R*)-1-(((*R*)-1-(1,3,6,2-dioxazaborolan-2-yl)-3-methylbutyl) amino)-3-(methylthio)-1-oxoprop-2-yl)-2,5-dichlorobenzamide, wherein the X-ray powder diffraction pattern of the crystal form B has characteristic peaks at the following diffraction angles 2θ: 8.44, 14.39, 15.08, 16.98, 19.76, 22.17, 22.41, 22.53, and 25.62, the measurement error of 2θ being ±0.2.

Provided is a method for preparing the crystal form B, which is as follows:
step (1): dissolving the crystal form A according to claim 1 in an organic solvent III and stirring at 60 °C for complete dissolving;
step (2), adding a proper amount of a solvent IV dropwise to the solution obtained in step (1), crystallizing, filtering, and drying to obtain the crystal form B;

wherein, the solvent III and the solvent IV are independently selected from dimethylformamide, petroleum ether, ketone solvents, ether solvents, alcohol solvents, nitrile solvents, or the mixture thereof;
the volume-to-mass ratio of the solvent III to the raw material crystal form A is 5-30:1 and is expressed in mL/g;
the volume-to-mass ratio of the solvent IV to the raw material crystal form A is 80-120:1 and is expressed in mL/g.

Provide is a crystal form D of *N*-((*R*)-1-(((*R*)-1-(1,3,6,2-dioxazaborolan-2-yl)-3-methylbutyl) amino)-3-(methylthio)-1-oxoprop-2-yl)-2,5-dichlorobenzamide, wherein the X-ray powder diffraction pattern of the crystal form D has characteristic peaks at the following diffraction angles 2θ: 6.88, 8.26, 9.15, 11.47, 13.82, 16.61, 18.39, 19.64, and 20.80, the measurement error of 2θ being ±0.2.

Provided is a method for preparing the crystal form D, which comprises the following steps:
step (1): dissolving the crystal form A according to claim 1 in an organic solvent V, stirring at 60 °C for 5 h, and crystallizing;
step (2): filtering and drying to obtain the crystal form D;
wherein, the solvent V is selected from dimethylformamide, petroleum ether, ketone solvents, ether solvents, alcohol solvents, nitrile solvents, or the mixture thereof; further, the volume-to-mass ratio of the solvent V to the raw material crystal form A is 5-15:1 and is expressed in mL/g.

The present invention also provides a pharmaceutical composition, which comprises a pharmaceutically acceptable carrier and one crystal or a combination of two or more crystals selected from the group consisting of:
(a) the crystal form A of the present invention;
(b) the crystal form B of the present invention;
(c) the crystal form D of the present invention.

The crystal form A, the crystal form B, or the crystal form D of the compound of formula (I), *N-*((*R*)-1-(((*R*)-1-(1,3,6,2-dioxazaborolan-2-yl)-3-methylbutyl)amino)-3-(methylthio)-1-oxoprop-2 -yl)-2,5-dichlorobenzamide, disclosed in the present invention can be used for preparing a medicament for preventing or treating solid tumors and hematological tumors. The solid tumors include non-small cell lung cancer, small cell lung cancer, lung adenocarcinoma, squamous cell lung cancer, pancreatic cancer, mammary cancer, prostatic cancer, liver cancer, skin cancer, epithelial cell cancer, gastrointestinal stromal tumor, or nasopharyngeal carcinoma. The hematological tumor-related diseases include leukemia, multiple myeloma, mantle cell lymphoma, or histiocytic lymphoma.

The crystal form A, the crystal form B, or the crystal form D of the compound of formula (I) disclosed in the present invention can be administered alone or in combination with other drugs that can effectively treat the diseases for preventing and treating solid tumors and hematological tumor-related diseases.

The present disclosure also provides a pharmaceutical composition for preventing or treating solid tumors and hematological tumor-related diseases, which comprises a therapeutically effective amount of the crystal form A, the crystal form B, or the crystal form D of the compound of formula (I) disclosed in the present invention and a pharmaceutically acceptable carrier. The pharmaceutical composition may be a common tablet, sustained-release tablet, controlled-release tablet, capsule, granule, pulvis, oral liquid, or injection.

The three crystal forms provided in the present invention have relatively high solubility and good stability, are beneficial to long-term storage and placement of drugs, and are suitable for preparing drug preparations; the preparation methods are simple and feature good repeatability and high yield, the concentration operation is avoided, and the clarity of the product is high; the process is simple and efficient, can realize large-scale production, and is environment-friendly.

### BRIEF DESCRIPTION OF THE DRAWINGS

FIG. 1 shows the X-ray powder diffraction pattern of the white eutectic solid sample obtained in Example 1.
FIG. 2 shows the X-ray powder diffraction pattern of the crystal form A, the product obtained in Example 1.
FIG. 3 shows the DSC pattern of the crystal form A, the product obtained in Example 1.
FIG. 4 shows the infrared spectrum of the crystal form A, the product obtained in Example 1.
FIG. 5 shows the X-ray powder diffraction pattern of the product, the crystal form B, obtained in Example 2.
FIG. 6 shows the DSC pattern of the crystal form B, the product obtained in Example 2.
FIG. 7 shows the infrared spectrum of the crystal form B, the product obtained in Example 2.
FIG. 8 shows the X-ray powder diffraction pattern of the crystal form D, the product obtained in Example 3.
FIG. 9 shows the DSC pattern of the crystal form D, the product obtained in Example 3.
FIG. 10 shows the infrared spectrum of the crystal form D, the product obtained in Example 3.

### DETAILED DESCRIPTION

The present invention is further described below with reference to specific examples according to the general technical knowledge and conventional practice in the art. The following examples are only some of the preferred examples of the present invention and should not be construed as limiting the present invention, and it is apparent to those skilled in the art that several modifications can be made without departing from the scope of the present invention and these modifications shall also fall within the protection scope of the present invention as defined by the claims.

### Example 1:

### Preparation of Crystal Form A

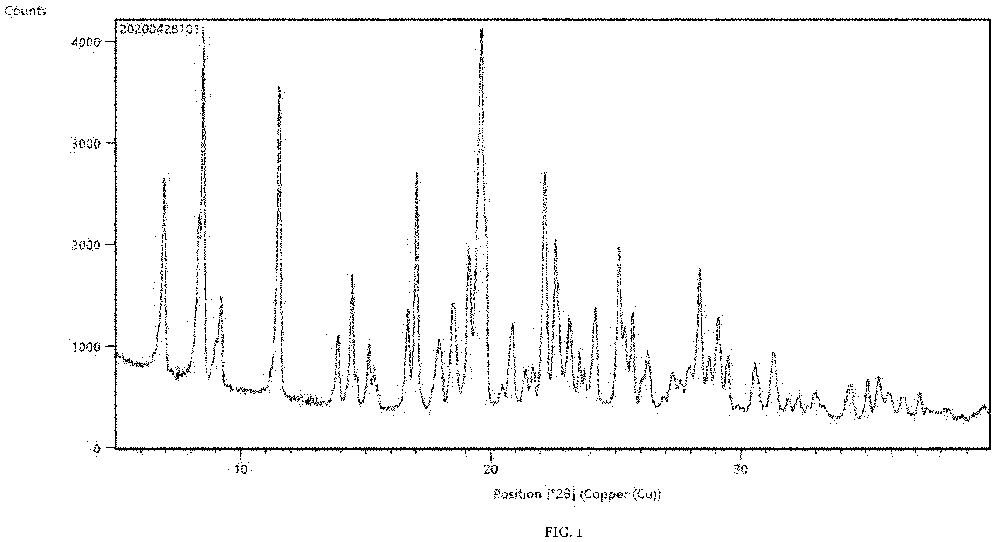

40 g of diethanolamine and 1.20 L of ethyl acetate were added to a reaction vessel, and the system was heated to 70 °C for dissolving. The temperature of the system was maintained at 70 °C, and the ((*R*)-1-((*R*)-2-(2,5-dichlorobenzamido)-3-(methylthio)propionamido)-3-methylbutyl)boric acid/ethyl acetate solution (dissolving 150 g of ((*R*)-1-((*R*)-2-(2,5-dichlorobenzamido)-3-(methylthio) propionamido)-3-methylbutyl)boric acid in 0.3 L of ethyl acetate solution) was added dropwise. After the dropwise addition, the system was cooled to 60 °C and stirred at 60 °C for 2 h, and then the system was cooled to 25 °C and stirred for 8 h while maintaining the temperature at 25 °C. The system was filtered, and the filter cake was dried to obtain 147 g of a white eutectic solid, and the X-ray powder diffraction pattern is shown in FIG. 1.

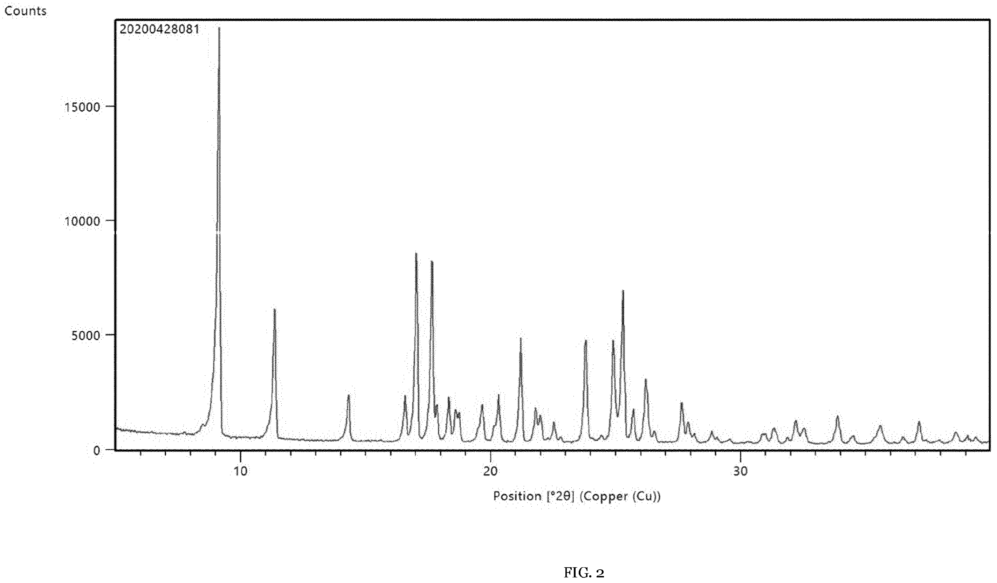

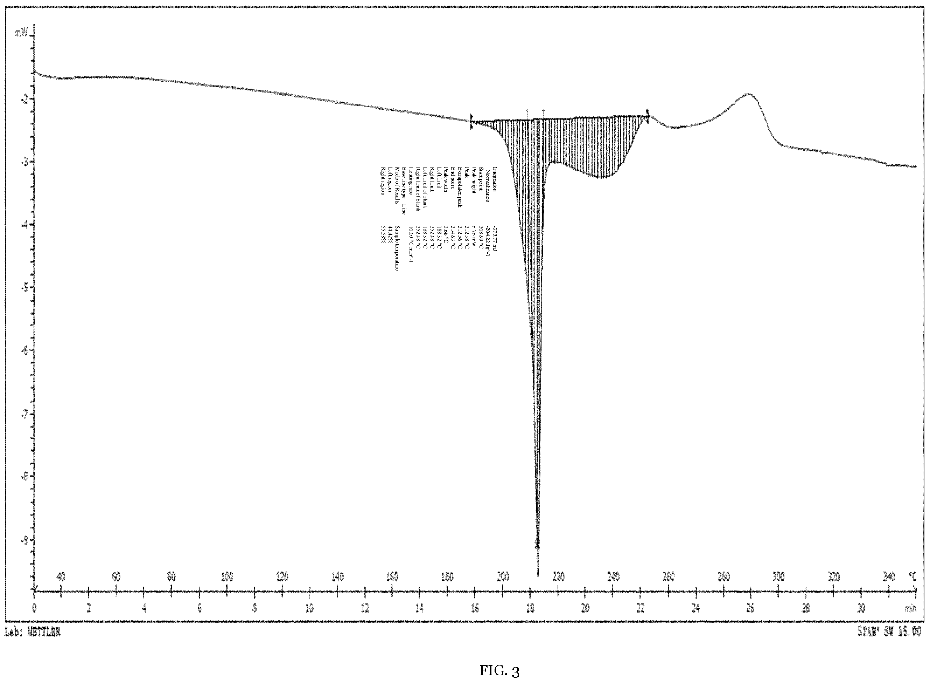

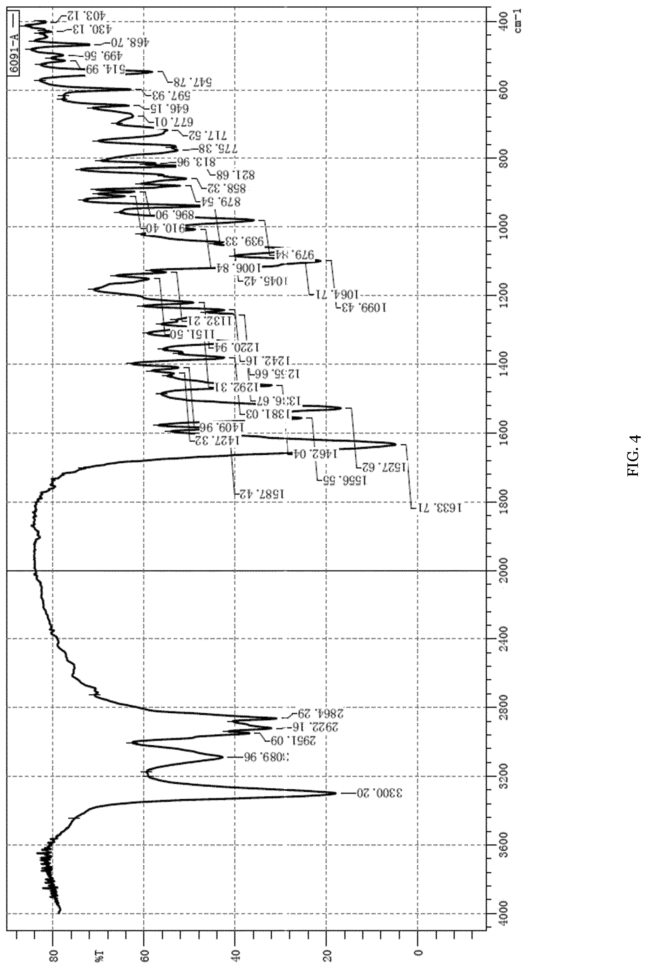

5 g of the eutectic solid sample obtained above and 50 mL of acetonitrile were added to a reaction vessel, and the system was stirred for 2 h at 30 °C. Then the system was heated to 60 °C with a heating rate of 15 °C/h and slurried for 2 h, and then the system was cooled to 30 °C with a cooling rate of 15 °C/h and slurried for 2 h. The system was filtered and dried at 35 °C to obtain 4.8 g of a white solid, namely the crystal form A. The X-ray powder diffraction pattern is shown in FIG. 2, the DSC pattern is shown in FIG. 3, and the infrared spectrum is shown in FIG. 4.

### Example 2:

### Preparation of Crystal Form B

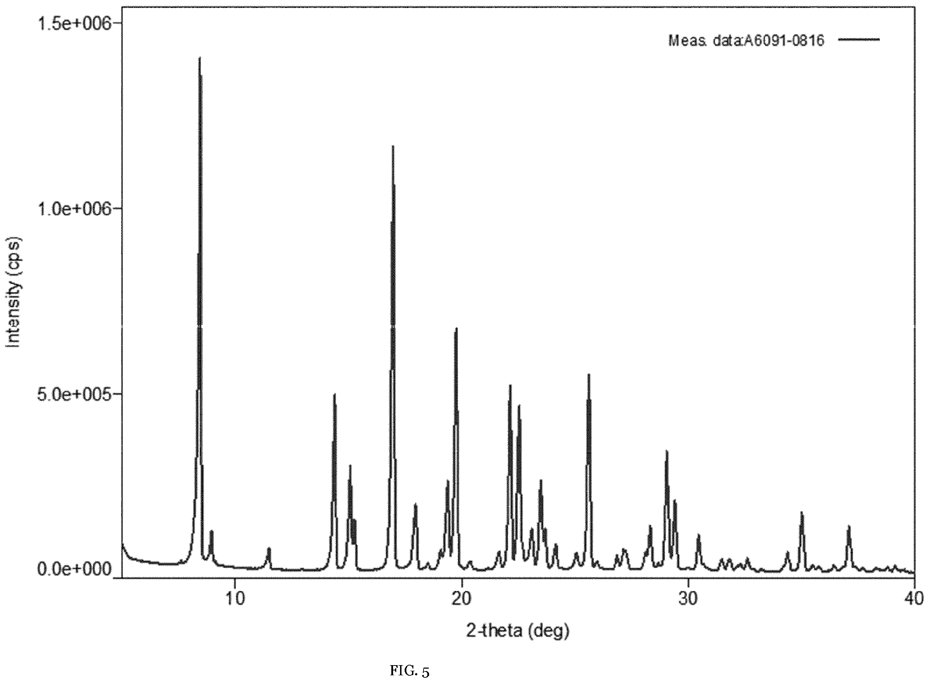

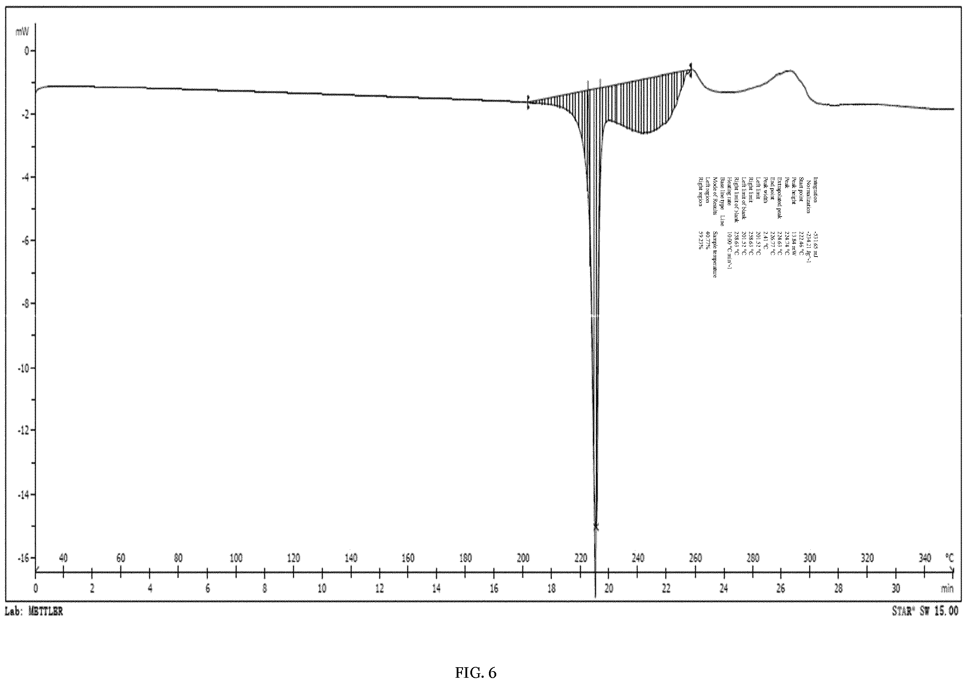

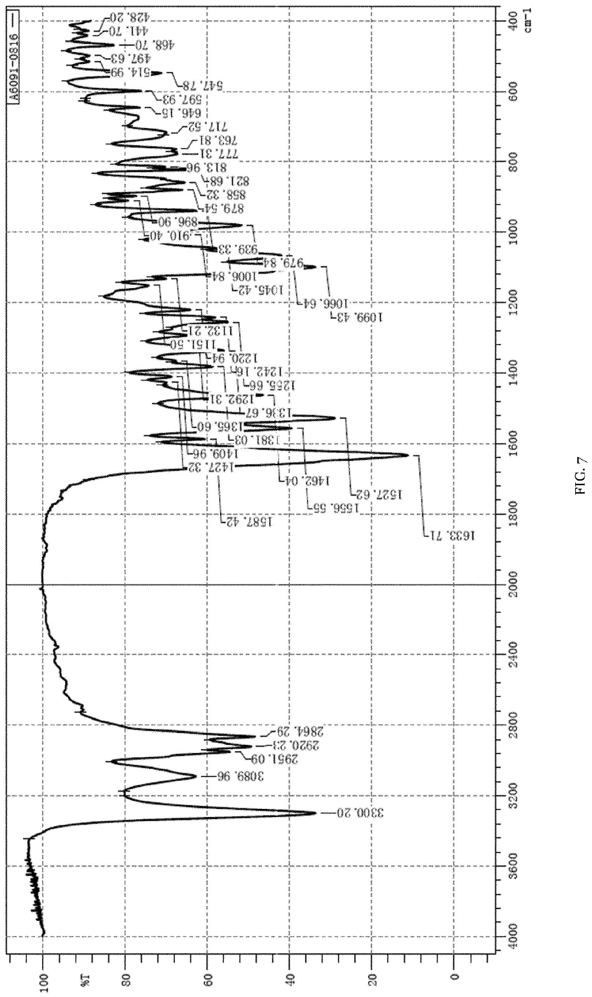

1 g of the crystal form A sample and 20 mL of *N,N-*dimethylformamide were added to a reaction vessel, and the system was heated to 65 °C for dissolving. 100 mL of petroleum ether was added dropwise, followed by crystallization. The system was dried at 35 °C to obtain 0.72 g of a white solid, namely the crystal form B. The X-ray powder diffraction pattern is shown in FIG. 5, the DSC pattern is shown in FIG. 6, and the infrared spectrum is shown in FIG. 7.

### Example 3:

### Preparation of Crystal Form D

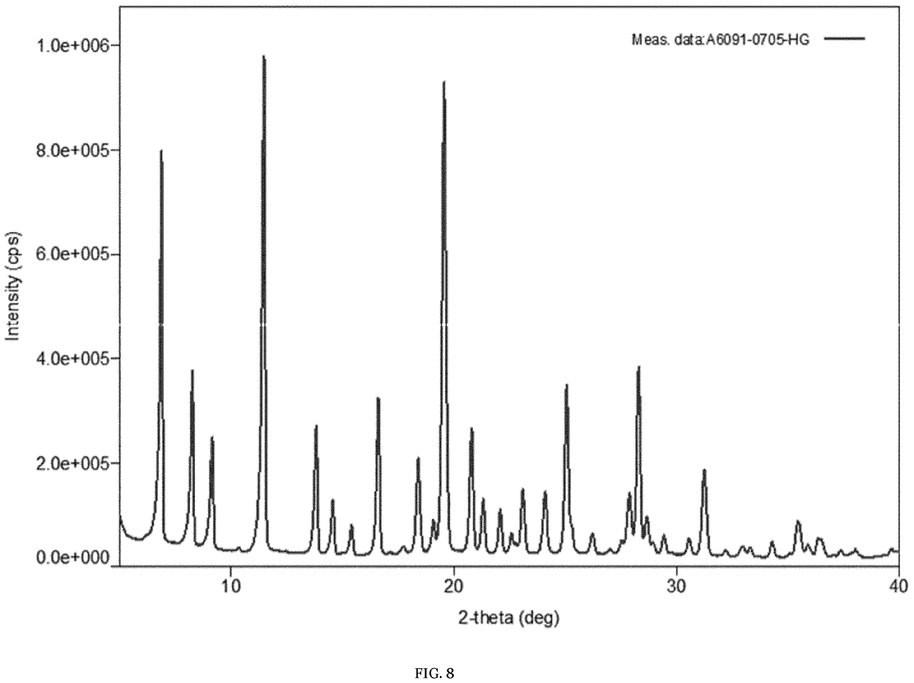

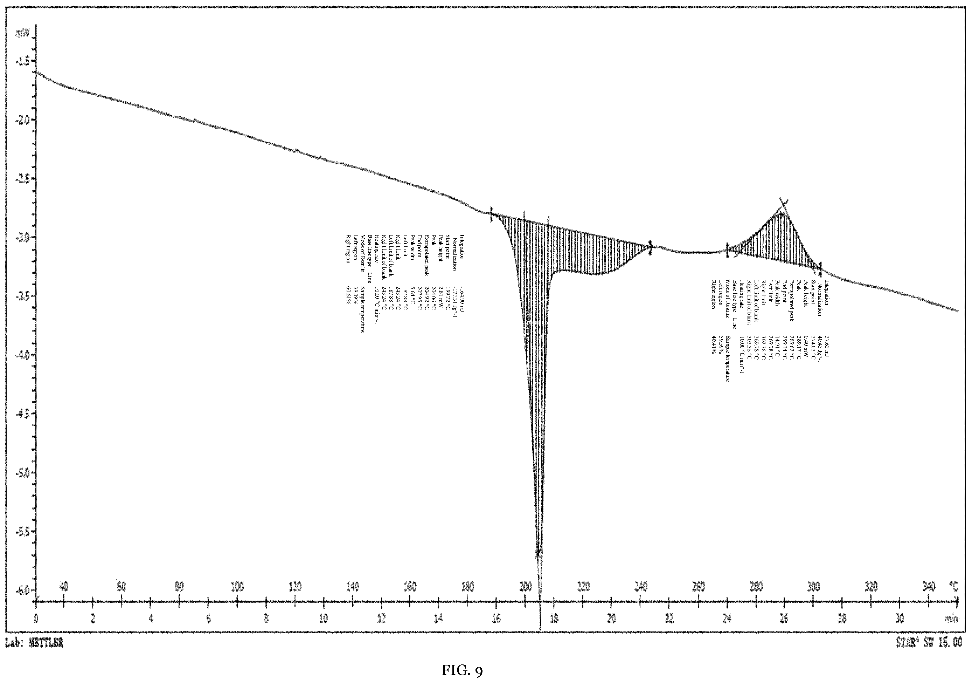

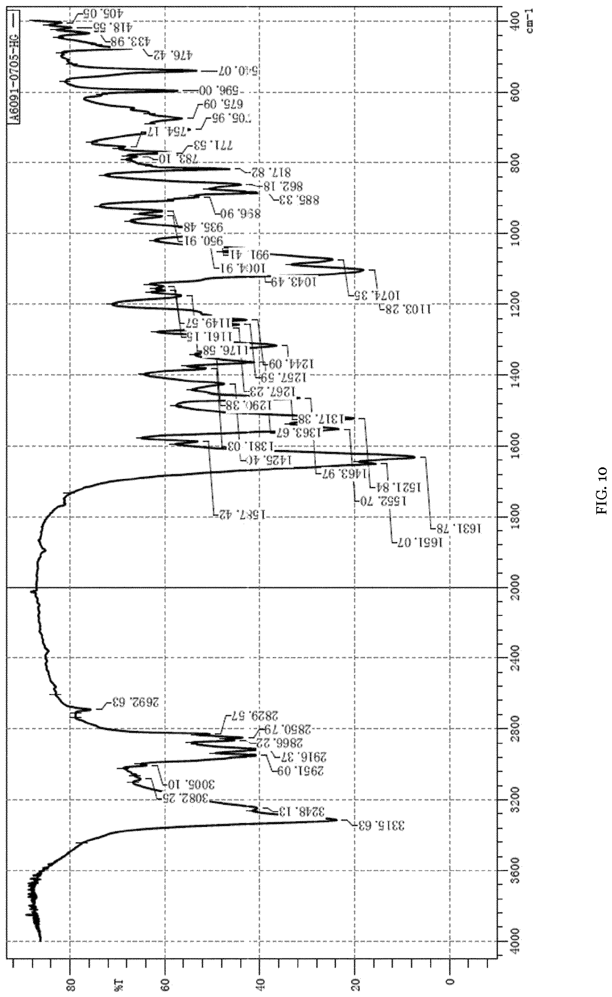

1 g of the crystal form A sample and 10 mL of tetrahydrofuran were added to a reaction vessel, and the system was heated to 60 °C and stirred for 5 h. Then the system was filtered and dried at 25 °C to obtain 0.85 g of a white solid, namely the crystal form D. The X-ray powder diffraction pattern is shown in FIG. 8, the DSC pattern is shown in FIG. 9, and the infrared spectrum is shown in FIG. 10.

### Example 4:

Four crystal samples produced, namely the eutectic sample and the crystal form A sample of Example 1, the crystal form B sample of Example 2, and the crystal form D sample of Example 3, were subjected to solubility tests and a kinetic solubility determination tests in ultra-pure water, and the solubility results are shown in Tables 1 and 2. It can be seen that the eutectic and the crystal forms A, B, and D of the compound (I) exhibit excellent solubility property.

**Table 1. Solubility of four crystal forms of Examples 1, 2, and 3 in ultra-pure water**

| Crystal forms of Examples | Dissolving medium | Upper limit (µg/mL) | Solubility (µg/mL) |
|---|---|---|---|
| White eutectic solid of Example 1 | Ultra-pure water | 300 | 310.12 |
| Crystal form A of Example 1 | Ultra-pure water | 300 | 318.09 |
| Crystal form B of Example 2 | Ultra-pure water | 300 | 302.80 |
| Crystal form D of Example 3 | Ultra-pure water | 300 | 304.21 |

**Table 2. Kinetic solubility of four crystal forms of Examples 1, 2, and 3 in ultra-pure water**

| Crystal forms of Examples | Dissolving medium | Upper limit (µg/mL) | Solubility (µg/mL) |
|---|---|---|---|
| White eutectic solid of Example 1 | Ultra-pure water | 1000 | 948.16 |
| Crystal form A of Example 1 | Ultra-pure water | 1000 | 958.47 |
| Crystal form B of Example 2 | Ultra-pure water | 1000 | 1078.59 |
| Crystal form D of Example 3 | Ultra-pure water | 1000 | 902.91 |

| | | | |
|---|---|---|---|
| Note: any value near or above the upper limit indicates that the solubility of the compound may reach or exceed the upper limit. | | | |

### Example 5:

Four crystal samples produced, namely the eutectic sample and the crystal form A sample of Example 1, the crystal form B sample of Example 2, and the crystal form D sample of Example 3, were subjected to hygroscopicity tests at 25 °C under a changing relative humidity of 0-80% RH. It can be seen that the crystal forms A and B of the compound (I) exhibit weaker hygroscopicity compared with the crystal form D and are more suitable for the preparation of solid preparations.

**Table 3. Results of dynamic vapor sorption determination of four crystal forms of Examples 1, 2, and 3**

| Crystal forms of Examples | Weight change in the range of 0% RH to 80% RH | Hygroscopicity |
|---|---|---|
| White eutectic solid of Example 1 | 2.1% | Deliquescent |
| Crystal form A of Example 1 | 1.4% | Slightly hygroscopic |
| Crystal form B of Example 2 | Less than 0.2% | Nonhygroscopic |
| Crystal form D of Example 3 | 2.4% | Deliquescent |

### Example 6: Stability Tests of Different Crystal Forms of Compound (I)

Four crystal samples produced, namely the eutectic sample and the crystal form A sample of Example 1, the crystal form B sample of Example 2, and the crystal form D sample of Example 3, were each subjected the stability influence factor tests under high temperature (60 °C, in the dark), high humidity (relative humidity of 90%±5%, in the dark), and illumination (45001x±5001x), and sampling was carried out to on day 0, day 5 day, and day 10 for HPLC determination, and the results are shown in Table 4 below. During the 10 days of examination, the eutectic sample of Example 1 exhibited significant content change under high humidity, and the eutectic sample was unstable. During the 10 days of examination, the crystal form A, B, and D samples showed no significant content change, the crystal forms didn't change, and the quality was stable.

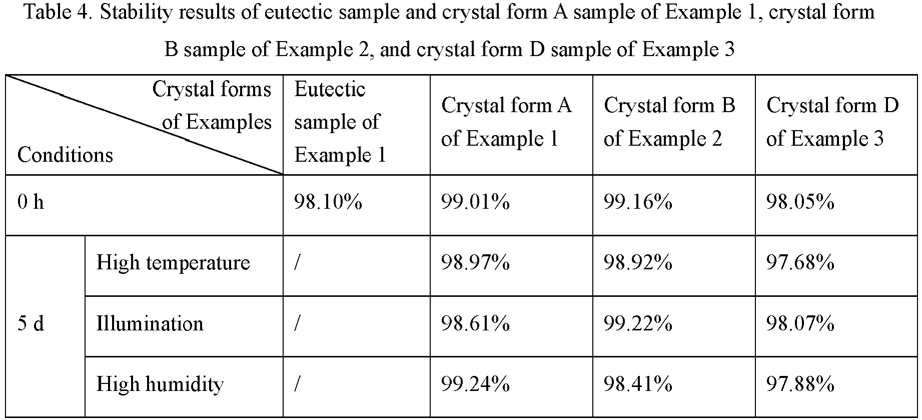

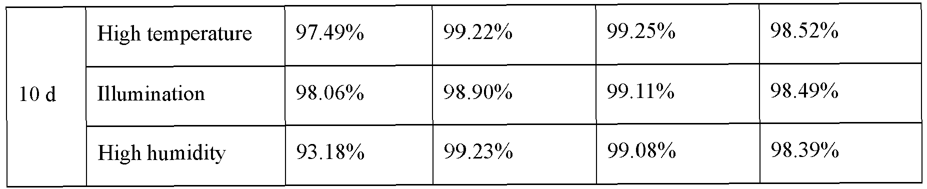

## Claims

1. A crystal form A of *N*-((*R*)-1-(((*R*)-1-(1,3,6,2-dioxazaborolan-2-yl)-3-methylbutyl)amino)-3-(methylthio)-1-oxoprop-2-yl)-2,5-dichlorobenzamide, wherein the X-ray powder diffraction pattern of the crystal form A has characteristic peaks at the following diffraction angles 2θ: 9.09, 11.30, 14.27, 17.00, 17.61, 20.28, 23.75, 24.85, and 25.25, the measurement error of 2θ being ±0.2.

2. The crystal form A according to claim 1, wherein the crystal form A is prepared mainly by the following steps:
step (1): dissolving an organic base in an organic solvent I and heating to 70-75 °C;
step (2): adding ((R)-1-((R)-2-(2,5-dichlorobenzamido)-3-(methylthio)propionamido)-3-methylbutyl)boric acid/organic solvent I solution dropwise to the solution obtained in step (1) while maintaining the temperature at 70-75 °C;
step (3): after the dropwise addition, cooling to 60 °C, stirring for 2 h, cooling to 20-25 °C, stirring for 8 h, filtering, and drying to obtain a eutectic solid;
step (4): dissolving the eutectic solid in an organic solvent II, stirring for 2 h at 25-35 °C, heating to 55-65 °C and stirring for 2 h with a heating rate of 15 °C/h, stirring for 2 h at 25-35 °C with a cooling rate of 15 °C/h, and crystallizing;
step (5): filtering and drying to obtain the crystal form A;
wherein, the organic base is selected from diethanolamine, ethanolamine, or ethylenediamine;
the solvent I or II is selected from ethyl acetate, dichloromethane, ketone solvents, ether solvents, alcohol solvents, nitrile solvents, or mixtures thereof;
the volume-to-mass ratio of the solvent I to the organic base is 15-45:1 and is expressed in mL/g;
the volume-to-mass ratio of the solvent II to the eutectic solid is 5-15:1 and is expressed in mL/g.

3. A crystal form B of *N*-((*R*)-1-(((*R*)-1-(1,3,6,2-dioxazaborolan-2-yl)-3-methylbutyl)amino)-3-(methylthio)-1-oxoprop-2-yl)-2,5-dichlorobenzamide, wherein the X-ray powder diffraction pattern of the crystal form B has characteristic peaks at the following diffraction angles 2θ: 8.44, 14.39, 15.08, 16.98, 19.76, 22.17, 22.41, 22.53, and 25.62, the measurement error of 2θ being ±0.2.

4. A method for preparing the crystal form B according to claim 3, wherein the crystal form B is prepared mainly by the following steps:
step (1): dissolving the crystal form A according to claim 1 in an organic solvent III and stirring at 60 °C for complete dissolving;
step (2), adding a proper amount of a solvent IV dropwise to the solution obtained in step (1), crystallizing, filtering, and drying to obtain the crystal form B;
wherein, the solvent III and the solvent IV are independently selected from dimethylformamide, petroleum ether, ketone solvents, ether solvents, alcohol solvents, nitrile solvents, or the mixture thereof;
the volume-to-mass ratio of the solvent III to the raw material crystal form A is 5-30:1 and is expressed in mL/g;
the volume-to-mass ratio of the solvent IV to the raw material crystal form A is 80-120:1 and is expressed in mL/g.

5. A crystal form D of *N*-((*R*)-1-(((*R*)-1-(1,3,6,2-dioxazaborolan-2-yl)-3-methylbutyl)amino)-3-(methylthio)-1-oxoprop-2-yl)-2,5-dichlorobenzamide, wherein the X-ray powder diffraction pattern of the crystal form D has characteristic peaks at the following diffraction angles 2θ: 6.88, 8.26, 9.15, 11.47, 13.82, 16.61, 18.39, 19.64, and 20.80, the measurement error of 2θ being ±0.2.

6. The crystal form D according to claim 5, wherein the crystal form D is prepared mainly by the following steps:
step (1): dissolving the crystal form A according to claim 1 in an organic solvent V, stirring at 60 °C for 5 h, and crystallizing;
step (2): filtering and drying to obtain the crystal form D;
wherein, the solvent V is selected from dimethylformamide, petroleum ether, ketone solvents, ether solvents, alcohol solvents, nitrile solvents, or the mixture thereof;
the volume-to-mass ratio of the solvent V to the raw material crystal form A is 5-15:1 and is expressed in mL/g.

7. A pharmaceutical composition, comprising a pharmaceutically acceptable carrier and one crystal or a combination of two or more crystals selected from the group consisting of:
(a) the crystal form A according to claim 1 or 2;
(b) the crystal form B according to claim 3 or 4;
(c) the crystal form D according to claim 5 or 6.

8. A method for preparing the crystal form A according to claim 1, comprising the following steps:
step (1): dissolving an organic base in an organic solvent I and heating to 70-75 °C;
step (2): adding ((*R*)-1-((*R*)-2-(2,5-dichlorobenzamido)-3-(methylthio)propionamido)-3-methylbutyl)boric acid/organic solvent I solution dropwise to the solution obtained in step (1) while maintaining the temperature at 70-75 °C;
step (3): after the dropwise addition, cooling to 60 °C, stirring for 2 h, cooling to 20-25 °C, stirring for 8 h, filtering, and drying to obtain a eutectic solid;
step (4): dissolving the eutectic solid in an organic solvent II, stirring for 2 h at 25-35 °C, heating to 55-65 °C and stirring for 2 h with a heating rate of 15 °C/h, stirring for 2 h at 25-35 °C with a cooling rate of 15 °C/h, and crystallizing;
step (5): filtering and drying to obtain the crystal form A;
wherein, the organic base is selected from diethanolamine, ethanolamine, or ethylenediamine;
the solvent I or II is selected from ethyl acetate, dichloromethane, ketone solvents, ether solvents, alcohol solvents, nitrile solvents, or mixtures thereof;
the volume-to-mass ratio of the solvent I to the organic base is 15-45:1 and is expressed in mL/g;
the volume-to-mass ratio of the solvent II to the eutectic solid is 5-15:1 and is expressed in mL/g.

9. The method according to claim 4, comprising the following steps:
step (1): dissolving the crystal form A according to claim 1 in an organic solvent III and stirring at 60 °C for complete dissolving;
step (2), adding a proper amount of a solvent IV dropwise to the solution obtained in step (1), crystallizing, filtering, and drying to obtain the crystal form B;
wherein, the solvent III and the solvent IV are independently selected from dimethylformamide, petroleum ether, ketone solvents, ether solvents, alcohol solvents, nitrile solvents, or the mixture thereof;
the volume-to-mass ratio of the solvent III to the raw material crystal form A is 10-30:1 and is expressed in mL/g;
the volume-to-mass ratio of the solvent IV to the raw material crystal form A is 80-120:1 and is expressed in mL/g.

10. A method for preparing the crystal form D according to claim 5, comprising the following steps:
step (1): dissolving the crystal form A according to claim 1 in an organic solvent V, stirring at 60 °C for 5 h, and crystallizing;
step (2): filtering and drying to obtain the crystal form D;
wherein, the solvent V is selected from dimethylformamide, petroleum ether, ketone solvents, ether solvents, alcohol solvents, nitrile solvents, or the mixture thereof;
the volume-to-mass ratio of the solvent V to the raw material crystal form A is 5-15:1 and is expressed in mL/g.

## Patentansprüche

1. Kristallform A von *N*-((*R*)-1-(((*R*)-1-(1,3,6,2-dioxazaborolan-2-yl)-3-methylbutyl)amino)-3-(methylthio)-1-oxoprop-2-yl)-2,5-dichlorbenzamid, wobei das Röntgenpulverdiffraktionsmuster der Kristallform A charakteristische Peaks bei den folgenden Beugungswinkeln 2θ aufweist: 9,09, 11,30, 14,27, 17,00, 17,61, 20,28, 23,75, 24,85 und 25,25, wobei der Messfehler von 2θ ±0,2 beträgt.

2. Kristallform A nach Anspruch 1, wobei die Kristallform A hauptsächlich durch die folgenden Schritte hergestellt wird:
Schritt (1): Auflösen einer organischen Base in einem organischen Lösungsmittel I und Erhitzen auf 70-75 °C;
Schritt (2): Tropfenweises Hinzufügen von (*R*)-1-((*R*)-2-(2,5-Dichlorbenzamido)-3-(methylthio)propionamido)-3-methylbutyl)borsäure/organisches Lösungsmittel I-Lösung zu der in Schritt (1) erhaltenen Lösung, während die Temperatur bei 70-75 °C gehalten wird;
Schritt (3): Nach dem tropfenweisen Hinzufügen, Abkühlen auf 60 °C, Rühren für 2 h, Abkühlen auf 20-25 °C, Rühren für 8 h, Filtrieren und Trocknen, um einen eutektischen Feststoff zu erhalten;
Schritt (4): Auflösen des eutektischen Feststoffs in einem organischen Lösungsmittel II, Rühren für 2 h bei 25-35 °C, Erhitzen auf 55-65 °C und Rühren für 2 h mit einer Heizrate von 15 °C/h, Rühren für 2 h bei 25-35 °C mit einer Abkühlrate von 15 °C/h, und Kristallisieren;
Schritt (5): Filtrieren und Trocknen, um die Kristallform A zu erhalten;
wobei die organische Base aus Diethanolamin, Ethanolamin oder Ethylendiamin ausgewählt ist;
das Lösungsmittel I oder II aus Ethylacetat, Dichlormethan, Ketonlösungsmitteln, Etherlösungsmitteln, Alkoholösungsmitteln, Nitrillösungsmitteln oder Mischungen davon ausgewählt ist;
das Volumen-zu-Masse-Verhältnis des Lösungsmittels I zur organischen Base 15-45:1 ist und in mL/g ausgedrückt wird;
das Volumen-zu-Masse-Verhältnis des Lösungsmittels II zum eutektischen Feststoff 5-15:1 ist und in mL/g ausgedrückt wird.

3. Kristallform B von *N*-((*R*)-1-(((*R*)-1-(1,3,6,2-dioxazaborolan-2-yl)-3-methylbutyl)amino)-3-(methylthio)-1-oxoprop-2-yl)-2,5-dichlorbenzamid, wobei das Röntgenpulverdiffraktionsmuster der Kristallform B charakteristische Peaks bei den folgenden Beugungswinkeln 2θ aufweist: 8,44, 14,39, 15,08, 16,98, 19,76, 22,17, 22,41, 22,53, und 25,62, wobei der Messfehler von 2θ ±0,2 beträgt.

4. Verfahren zur Herstellung der Kristallform B nach Anspruch 3, wobei die Kristallform B hauptsächlich durch die folgenden Schritte hergestellt wird:
Schritt (1): Auflösen der Kristallform A nach Anspruch 1 in einem organischen Lösungsmittel III und Rühren bei 60 °C zum vollständigen Auflösen;
Schritt (2), tropfenweises Hinzufügen einer geeigneten Menge eines Lösungsmittels IV zu der in Schritt (1) erhaltenen Lösung, Kristallisieren, Filtrieren und Trocknen, um die Kristallform B zu erhalten;
wobei das Lösungsmittel III und das Lösungsmittel IV unabhängig voneinander ausgewählt werden aus Dimethylformamid, Petrolether, Ketonlösungsmitteln,
Etherlösungsmitteln, Alkohollösungsmitteln, Nitrillösungsmitteln oder Mischungen davon;
das Volumen-zu-Masse-Verhältnis des Lösungsmittels III zur Ausgangsstoff-Kristallform A 5-30: 1 ist und in mL/g ausgedrückt wird;
das Volumen-zu-Masse-Verhältnis des Lösungsmittels IV zur Ausgangsstoff-Kristallform A 80-120:1 ist und in mL/g ausgedrückt wird.

5. Kristallform D von *N*-((*R*)-1-(((*R*)-1-(1,3,6,2-dioxazaborolan-2-yl)-3-methylbutyl)amino)-3-(methylthio)-1-oxoprop-2-yl)-2,5-dichlorbenzamid, wobei das Röntgenpulverdiffraktionsmuster der Kristallform D charakteristische Peaks bei den folgenden Beugungswinkeln 2θ aufweist: 6,88, 8,26, 9,15, 11,47, 13,82, 16,61, 18,39, 19,64, und 20,80, wobei der Messfehler von 2θ ±0,2 beträgt.

6. Kristallform D nach Anspruch 5, wobei die Kristallform D hauptsächlich durch die folgenden Schritte hergestellt wird:
Schritt (1): Auflösen der Kristallform A nach Anspruch 1 in einem organischen Lösungsmittel V, Rühren bei 60 °C für 5 h und Kristallisieren;
Schritt (2): Filtrieren und Trocknen, um die Kristallform D zu erhalten;
wobei das Lösungsmittel V ausgewählt ist aus Dimethylformamid, Petrolether, Ketonlösungsmitteln, Etherlösungsmitteln, Alkohollösungsmitteln, Nitrillösungsmitteln oder Mischungen davon;
das Volumen-zu-Masse-Verhältnis des Lösungsmittels V zur Ausgangsstoff-Kristallform A 5-15:1 ist und in mL/g ausgedrückt wird.

7. Pharmazeutische Zusammensetzung, umfassend einen pharmazeutisch akzeptablen Träger und einen Kristall oder eine Kombination aus zwei oder mehr Kristallen, ausgewählt aus der Gruppe, bestehend aus:
(a) der Kristallform A nach Anspruch 1 oder 2;
(b) der Kristallform B nach Anspruch 3 oder 4;
(c) der Kristallform D nach Anspruch 5 oder 6.

8. Verfahren zur Herstellung der Kristallform A nach Anspruch 1, umfassend die folgenden Schritte:
Schritt (1): Auflösen einer organischen Base in einem organischen Lösungsmittel I und Erhitzen auf 70-75 °C;
Schritt (2): Tropfenweises Hinzufügen von (*R*)-1-((*R*)-2-(2,5-Dichlorbenzamido)-3-(methylthio)propionamido)-3-methylbutyl)borsäure/ organisches Lösungsmittel I-Lösung zu der in Schritt (1) erhaltenen Lösung, während die Temperatur bei 70-75 °C gehalten wird;
Schritt (3): Nach dem tropfenweisen Hinzufügen, Abkühlen auf 60 °C, Rühren für 2 h, Abkühlen auf 20-25 °C, Rühren für 8 h, Filtrieren und Trocknen, um einen eutektischen Feststoff zu erhalten;
Schritt (4): Auflösen des eutektischen Feststoffs in einem organischen Lösungsmittel II, Rühren für 2 h bei 25-35 °C, Erhitzen auf 55-65 °C und Rühren für 2 h mit einer Heizrate von 15 °C/h, Rühren für 2 h bei 25-35 °C mit einer Abkühlrate von 15 °C/h, und Kristallisieren;
Schritt (5): Filtrieren und Trocknen, um die Kristallform A zu erhalten;
wobei die organische Base aus Diethanolamin, Ethanolamin oder Ethylendiamin ausgewählt ist;
das Lösungsmittel I oder II aus Ethylacetat, Dichlormethan, Ketonlösungsmitteln, Etherlösungsmitteln, Alkoholösungsmitteln, Nitrillösungsmitteln oder Mischungen davon ausgewählt ist;
das Volumen-zu-Masse-Verhältnis des Lösungsmittels I zur organischen Base 15-45:1 ist und in mL/g ausgedrückt wird;
das Volumen-zu-Masse-Verhältnis des Lösungsmittels II zum eutektischen Feststoff 5-15:1 ist und in mL/g ausgedrückt wird.

9. Verfahren zur Herstellung nach Anspruch 4, umfassend die folgenden Schritte:
Schritt (1): Auflösen der Kristallform A nach Anspruch 1 in einem organischen Lösungsmittel III und Rühren bei 60 °C zum vollständigen Auflösen;
Schritt (2), tropfenweises Hinzufügen einer geeigneten Menge eines Lösungsmittels IV zu der in Schritt (1) erhaltenen Lösung, Kristallisieren, Filtrieren und Trocknen, um die Kristallform B zu erhalten;
wobei das Lösungsmittel III und das Lösungsmittel IV unabhängig voneinander ausgewählt werden aus Dimethylformamid, Petrolether, Ketonlösungsmitteln, Etherlösungsmitteln, Alkohollösungsmitteln, Nitrillösungsmitteln oder Mischungen davon;
das Volumen-zu-Masse-Verhältnis des Lösungsmittels III zur Ausgangsstoff-Kristallform A 10-30:1 ist und in mL/g ausgedrückt wird;
das Volumen-zu-Masse-Verhältnis des Lösungsmittels IV zur Ausgangsstoff-Kristallform A 80-120:1 ist und in mL/g ausgedrückt wird.

10. Verfahren zur Herstellung der Kristallform D nach Anspruch 5, umfassend die folgenden Schritte:
Schritt (1): Auflösen der Kristallform A nach Anspruch 1 in einem organischen Lösungsmittel V, Rühren bei 60 °C für 5 h und Kristallisieren;
Schritt (2): Filtrieren und Trocknen, um die Kristallform D zu erhalten;
wobei das Lösungsmittel V ausgewählt ist aus Dimethylformamid, Petrolether, Ketonlösungsmitteln, Etherlösungsmitteln, Alkohollösungsmitteln, Nitrillösungsmitteln oder Mischungen davon;
das Volumen-zu-Masse-Verhältnis des Lösungsmittels V zur Ausgangsstoff-Kristallform A 5-15:1 ist und in mL/g ausgedrückt wird.

## Revendications

1. Une forme cristalline A de N-((R)-1-(((R)-1-(1,3,6,2-dioxazaborolan-2-yl)-3-méthylbutyl)amino)-3-(méthylthio)-1-oxoprop-2-yl)-2,5-dichlorobenzamide, laquelle présente un diagramme de diffraction des rayons X sur poudre ayant des pics caractéristiques aux angles de diffraction 2θ suivants : 9,09 ; 11,30 ; 14,27 ; 17,00 ; 17,61 ; 20,28 ; 23,75 ; 24,85 et 25,25, l'erreur de mesure de 2θ étant de ±0,2.

2. La forme cristalline A selon la revendication 1, laquelle est préparée principalement par les étapes suivantes :
étape (1) : dissolution d'une base organique dans un solvant organique I et chauffage à 70-75 °C ;
étape (2) : addition goutte à goutte d'une solution d'acide borique de ((R)-1-((R)-2-(2,5-dichlorobenzamido)-3-(méthylthio)propionamido)-3-méthylbutyl)/solvant organique I à la solution obtenue à l'étape (1) tout en maintenant la température à 70-75 °C ;
étape (3) : après l'addition goutte à goutte, refroidissement à 60 °C, agitation pendant 2 h, refroidissement à 20-25 °C, agitation pendant 8 h, filtration et séchage pour obtenir un solide eutectique ;
étape (4) : dissolution du solide eutectique dans un solvant organique II, agitation pendant 2 h à 25-35 °C, chauffage à 55-65 °C et agitation pendant 2 h avec une vitesse de chauffage de 15 °C/h, agitation pendant 2 h à 25-35 °C avec une vitesse de refroidissement de 15 °C/h, et cristallisation ;
étape (5) : filtration et séchage pour obtenir la forme cristalline A ;
dans lequel la base organique est choisie parmi la diéthanolamine, l'éthanolamine ou l'éthylènediamine ; le solvant I ou II est choisi parmi l'acétate d'éthyle, le dichlorométhane, les solvants cétoniques, les solvants éther, les solvants alcooliques, les solvants nitrile ou leurs mélanges ;
le rapport volume/masse du solvant I à la base organique est de 15-45:1 et s'exprime en mL/g ;
le rapport volume/masse du solvant II au solide eutectique est de 5-15:1 et s'exprime en mL/g.

3. Une forme cristalline B de N-((R)-1-(((R)-1-(1,3,6,2-dioxazaborolan-2-yl)-3-méthylbutyl)amino)-3-(méthylthio)-1-oxoprop-2-yl)-2,5-dichlorobenzamide, laquelle présente un diagramme de diffraction des rayons X sur poudre ayant des pics caractéristiques aux angles de diffraction 2θ suivants : 8,44 ; 14,39 ; 15,08 ; 16,98 ; 19,76 ; 22,17 ; 22,41 ; 22,53 et 25,62, l'erreur de mesure de 2θ étant de ±0,2.

4. Procédé de préparation de la forme cristalline B selon la revendication 3, lequel comprend principalement les étapes suivantes :
étape (1) : dissolution de la forme cristalline A selon la revendication 1 dans un solvant organique III et agitation à 60 °C pour dissolution complète ;
étape (2) : addition goutte à goutte d'une quantité appropriée d'un solvant IV à la solution obtenue à l'étape (1), cristallisation, filtration et séchage pour obtenir la forme cristalline B ;
lequel le solvant III et le solvant IV sont choisis indépendamment parmi le diméthylformamide, l'éther de pétrole, les solvants cétoniques, les solvants éther, les solvants alcooliques, les solvants nitrile ou leur mélange ;
le rapport volume/masse du solvant III à la matière première forme cristalline A est de 5-30:1 et s'exprime en mL/g ;
le rapport volume/masse du solvant IV à la matière première forme cristalline A est de 80-120:1 et s'exprime en mL/g.

5. Une forme cristalline D de N-((R)-1-(((R)-1-(1,3,6,2-dioxazaborolan-2-yl)-3-méthylbutyl)amino)-3-(méthylthio)-1-oxoprop-2-yl)-2,5-dichlorobenzamide, laquelle présente un diagramme de diffraction des rayons X sur poudre ayant des pics caractéristiques aux angles de diffraction 2θ suivants : 6,88 ; 8,26 ; 9,15 ; 11,47 ; 13,82 ; 16,61 ; 18,39 ; 19,64 et 20,80, l'erreur de mesure de 2θ étant de ±0,2.

6. La forme cristalline D selon la revendication 5, laquelle est préparée principalement par les étapes suivantes :
étape (1) : dissolution de la forme cristalline A selon la revendication 1 dans un solvant organique V, agitation à 60 °C pendant 5 h et cristallisation ;
étape (2) : filtration et séchage pour obtenir la forme cristalline D ;
lequel le solvant V est choisi parmi le diméthylformamide, l'éther de pétrole, les solvants cétoniques, les solvants éther, les solvants alcooliques, les solvants nitrile ou leur mélange ;
le rapport volume/masse du solvant V à la matière première forme cristalline A est de 5-15:1 et s'exprime en mL/g.

7. Une composition pharmaceutique, comprenant un excipient pharmaceutiquement acceptable et un cristal ou une combinaison d'au moins deux cristaux choisis dans le groupe constitué de :
(a) la forme cristalline A selon l'une quelconque des revendications 1 à 2 ;
(b) la forme cristalline B selon l'une quelconque des revendications 3 à 4 ;
(c) la forme cristalline D selon l'une quelconque des revendications 5 à 6.

8. Procédé de préparation de la forme cristalline A selon la revendication 1, comprenant les étapes suivantes :
étape (1) : dissolution d'une base organique dans un solvant organique I et chauffage à 70-75 °C ;
étape (2) : addition goutte à goutte d'une solution d'acide borique de ((R)-1-((R)-2-(2,5-dichlorobenzamido)-3-(méthylthio)propionamido)-3-méthylbutyl)/solvant organique I à la solution obtenue à l'étape (1) tout en maintenant la température à 70-75 °C ;
étape (3) : après l'addition goutte à goutte, refroidissement à 60 °C, agitation pendant 2 h, refroidissement à 20-25 °C, agitation pendant 8 h, filtration et séchage pour obtenir un solide eutectique ;
étape (4) : dissolution du solide eutectique dans un solvant organique II, agitation pendant 2 h à 25-35 °C, chauffage à 55-65 °C et agitation pendant 2 h avec une vitesse de chauffage de 15 °C/h, agitation pendant 2 h à 25-35 °C avec une vitesse de refroidissement de 15 °C/h, et cristallisation ;
étape (5) : filtration et séchage pour obtenir la forme cristalline A ; lequel la base organique est choisie parmi la diéthanolamine, l'éthanolamine ou l'éthylènediamine ;
le solvant I ou II est choisi parmi l'acétate d'éthyle, le dichlorométhane, les solvants cétoniques, les solvants éther, les solvants alcooliques, les solvants nitrile ou leurs mélanges ;
le rapport volume/masse du solvant I à la base organique est de 15-45:1 et s'exprime en mL/g ;
le rapport volume/masse du solvant II au solide eutectique est de 5-15:1 et s'exprime en mL/g.

9. Le procédé selon la revendication 4, comprenant les étapes suivantes:
étape (1) : dissolution de la forme cristalline A selon la revendication 1 dans un solvant organique III et agitation à 60 °C pour dissolution complète ;
étape (2) : addition goutte à goutte d'une quantité appropriée d'un solvant IV à la solution obtenue à l'étape (1), cristallisation, filtration et séchage pour obtenir la forme cristalline B ;
lequel le solvant III et le solvant IV sont choisis indépendamment parmi le diméthylformamide, l'éther de pétrole, les solvants cétoniques, les solvants éther, les solvants alcooliques, les solvants nitrile ou leur mélange ;
le rapport volume/masse du solvant III à la matière première forme cristalline A est de 10-30:1 et s'exprime en mL/g ;
le rapport volume/masse du solvant IV à la matière première forme cristalline A est de 80-120:1 et s'exprime en mL/g.

10. Procédé de préparation de la forme cristalline D selon la revendication 5, comprenant les étapes suivantes :
étape (1) : dissolution de la forme cristalline A selon la revendication 1 dans un solvant organique V, agitation à 60 °C pendant 5 h et cristallisation ;
étape (2) : filtration et séchage pour obtenir la forme cristalline D ;
lequel le solvant V est choisi parmi le diméthylformamide, l'éther de pétrole, les solvants cétoniques, les solvants éther, les solvants alcooliques, les solvants nitrile ou leur mélange ;
le rapport volume/masse du solvant V à la matière première forme cristalline A est de 5-15:1 et s'exprime en mL/g.
